# EUROPEAN PATENT APPLICATION

(11) **EP 4 122 494 A1**
(43) Date of publication of application: **25.01.2023**
(21) Application number: 21771901.2
(22) Date of filing: 07.01.2021
(51) Int. Cl.: A61K 45/00, A61P 35/00, A61P 43/00, C07D 473/38, C07K 14/47, C07D 498/18, C07D 519/04, C07D 249/18, C12Q 1/02, A61K 38/08, G01N 33/53, G01N 33/574, A61K 31/13, A61K 31/136, A61K 31/4192, A61K 31/475, A61K 31/52, A61K 31/537, G01N 27/62

(54) **METHOD FOR DETECTING INDICATOR OF T-CELL LYMPHOMA AND UTILIZATION THEREOF**

(30) Priority: 16.03.2020 JP 2020045564
(71) Applicant: Mandom Corporation, Osaka-shi, Osaka 540-8530 (JP); OSAKA UNIVERSITY, Suita-shi Osaka 565-0871 (JP); Public University Corporation Nagoya City University, Mizuho-ku Nagoya-shi Aichi 467-8601 (JP)
(72) Inventor: TORIYAMA, Manami, Suita-shi, Osaka 565-0871 (JP); FUJITA, Fumitaka, Osaka-shi, Osaka 540-8530 (JP); OKADA, Fumihiro, Osaka-shi, Osaka 540-8530 (JP); MORITA, Akimichi, Nagoya-city, Aichi 467-8601 (JP); NAKAMURA, Motoki, Nagoya-city, Aichi 467-8601 (JP)
(74) Representative: TBK
(86) International application number: PCT/JP2021/000344
(87) International publication number: WO 2021/186855

(57) **Abstract**

The present invention provides a novel method for detecting an indicator of T cell lymphoma. The method for detecting an indicator of T cell lymphoma includes the step of detecting acetylated tubulin in a T cell that has been collected from a subject.

## Description

### Technical Field

The present invention relates to a method for detecting an indicator of T cell lymphoma, and use thereof.

### Background Art

T cell lymphoma is caused by canceration of a T cell, which is a type of lymphocyte. Sites at which T cell lymphoma develops are roughly classified into two groups, i.e., a lymphoid tissue and an extralymphatic organ (extranodal organ). The lymphoid tissue is a tissue or organ that is responsible for an immune function such as elimination of a pathogen (e.g., bacteria or virus). Specific examples of the lymphoid tissue include a lymph node, a thymus present near the chest, a spleen, and a tonsil. Specific examples of the extralymphatic organ include bone marrow, lung, large intestine, small intestine, and skin. The lymphoid tissues and extralymphatic organs are distributed throughout the body, and therefore T cell lymphoma can occur anywhere throughout the body.

T cell lymphoma that occurs in the skin is referred to as cutaneous T cell lymphoma (CTCL). Specific examples of diseases included in CTCL include mycosis fungoides, Sezary syndrome, adult T cell leukemia lymphoma, primary cutaneous CD30 positive lymphoproliferative disorder (e.g., anaplastic large cell lymphoma), subcutaneous panniculitis-like T cell lymphoma, and the like.

Examples of diagnosis of T cell lymphoma include diagnosis by inspection of the lesion and biopsy of a tissue collected from the lesion. Specific examples of the biopsy include diagnosis based on genetic analysis (see Patent Literatures 1 and 2), and diagnosis based on a test for canceration of a T cell in a tissue collected from the lesion. Specific examples of a method for testing canceration of a T cell include a method of fluorescently staining CCR4, which is a marker molecule for a cancerized T cell (see Patent Literature 3). Studies have also been conducted to treat T cell lymphoma by medication targeting CCR4 positive cells using an anti-CCR4 antibody.

### Citation List

### [Patent Literature]

[Patent Literature 1]
   WO2014/178432
[Patent Literature 2]
   Published Japanese Translation of PCT International Application Tokuhyo No. 2005-518790
[Patent Literature 3]
   Published Japanese Translation of PCT International Application Tokuhyo No. 2013-517464

### Summary of Invention

### Technical Problem

Cancer cells vary in their malignancy as they variously change. If it is possible to detect cancer cells at various stages (e.g., early stage), it may be possible to facilitate treatment of cancer. Therefore, there has been a need to develop a new method for detecting an indicator for diagnosing T cell lymphoma.

An embodiment of the present invention is accomplished in view of the above issues, and an object thereof is to provide a novel method for detecting an indicator of T cell lymphoma.

According to the present invention, it is possible to provide a novel method for detecting an indicator of T cell lymphoma.

### Solution to Problem

As a result of diligent studies to attain the object, the inventors of the present invention have newly found that a large amount of acetylated tubulin is expressed in T cell lymphoma, and have accomplished the present invention.

That is, in order to attain the object, an embodiment of the present invention encompasses the following features:
[1] A method for detecting an indicator of T cell lymphoma in accordance with an aspect of the present invention includes the step of detecting acetylated tubulin in a T cell that has been collected from a subject.
[2] A test kit for T cell lymphoma in accordance with an aspect of the present invention includes an anti-acetylated tubulin antibody.
[3] A detection method in accordance with an aspect of the present invention is a method for detecting an indicator for evaluating an inhibitory effect on T cell lymphoma in a subject to which a medicament has been administered, the method including the step of detecting acetylated tubulin in a T cell that has been collected from the subject to which the medicament had been administered.
[4] An evaluation method in accordance with an aspect of the present invention is a method for evaluating whether or not a test sample is a substance having an inhibitory function against T cell lymphoma, the method including the steps of: bringing the test sample into contact with a T cell that has been collected from a subject suffering from T cell lymphoma; and detecting acetylated tubulin in the T cell which has been brought into contact with the test sample.
[5] A therapeutic agent for T cell lymphoma in accordance with an aspect of the present invention contains, as an active ingredient, an agent that inhibits aggregation of acetylated tubulin and/or an agent that inhibits acetylation of tubulin.

### Advantageous Effects of Invention

According to an aspect of the present invention, it is possible to provide a novel method for detecting an indicator of T cell lymphoma.

### Brief Description of Drawings

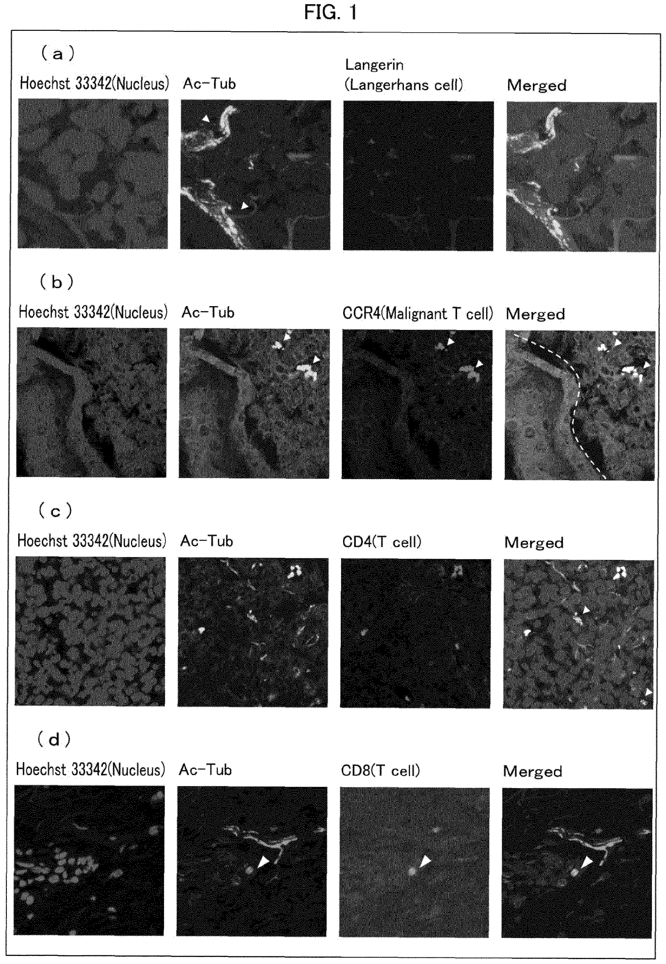

(a) through (d) in Fig. 1 show images for analyzing expression patterns of cell-surface markers for acetylated tubulin positive cells in accordance with an embodiment of the present invention.

### Description of Embodiments

The following description will discuss an embodiment of the present invention. The present invention is, however, not limited to the embodiment below. The present invention is not limited to features described below, but may be altered in various ways by a skilled person within the scope of the claims. The present invention also encompasses, in its technical scope, any embodiment and any working example derived by appropriately combining technical means disclosed in differing embodiments and working examples. Moreover, all literatures described in this specification are incorporated herein as reference literatures. Any numerical range "A to B" expressed in the present specification intends to mean "not less than A and not more than B".

### [1. Method for detecting indicator of T cell lymphoma]

The "method for detecting an indicator of T cell lymphoma" in accordance with an embodiment of the present invention can be "a method for detecting an indicator for diagnosing T cell lymphoma", can be "a method for obtaining data for diagnosing T cell lymphoma", or can be "an assistance method for diagnosing T cell lymphoma".

The method for detecting an indicator of T cell lymphoma in accordance with an embodiment of the present invention includes the step of detecting acetylated tubulin in a T cell that has been collected from a subject. Examples of the T cell lymphoma include mycosis fungoides, Sezary syndrome, adult T cell leukemia lymphoma, primary cutaneous CD30 positive lymphoproliferative disorder (e.g., anaplastic large cell lymphoma), subcutaneous panniculitis-like T cell lymphoma, and the like.

### (Subject)

The subject is not particularly limited, and examples thereof include a human and non-human animals. The non-human animals are not particularly limited, and examples thereof include experimental animals, pets, and domestic animals. More specifically, the non-human animals can be a mouse, a rat, a rabbit, a cat, a dog, a cow, a sheep, a goat, a pig, and a horse. The subject can be a tissue (e.g., skin tissue) collected from the foregoing human or non-human animal.

### (T cell)

The T cell in which acetylated tubulin is to be detected is not particularly limited, and examples thereof include a CD4⁺ T cell, a CCR4⁺ T cell, a CD8⁺ T cell, a CD4⁺CCR4⁺ T cell, a CD8⁺CCR4⁺ T cell, and a CD4⁺CD8⁺CCR4⁺ T cell. Note that CD4 and CD8 are surface antigens of T cells, and CCR4 is a chemokine receptor present on a T cell.

It is known that CCR4 is frequently expressed in high-malignancy-grade cancers. The T cell in which acetylated tubulin is to be detected can therefore be a CD4⁺ T cell, a CD8⁺ T cell, a CCR4⁺ T cell, a CD4⁺CCR4⁺ T cell, a CD8⁺CCR4⁺ T cell or a CD4⁺CD8⁺CCR4⁺ T cell. Early-stage T cell lymphoma can be diagnosed using a CD4⁺ T cell or a CD8⁺ T cell as a T cell in which acetylated tubulin is to be detected. Late-stage T cell lymphoma can be diagnosed using a CCR4⁺ T cell as a T cell in which acetylated tubulin is to be detected. Both early-stage T cell lymphoma and late-stage T cell lymphoma can be diagnosed using a CD4⁺CCR4⁺ T cell or a CD8⁺CCR4⁺ T cell as a T cell in which acetylated tubulin is to be detected.

A method of collecting a T cell from the foregoing subject is not particularly limited. For example, it is possible that a section of a tissue (e.g., skin tissue) is collected from the subject, and a T cell in a state of being contained in the tissue is collected.

### (Acetylated tubulin)

A microtubule is a cylindrical protein fiber formed primarily by a polymer of α-tubulin and β-tubulin. In the microtubule, polymerization and depolymerization of α-tubulin and β-tubulin occur constantly, and cell morphology and cell movement are controlled by balance of the polymerization and depolymerization.

A microtubule is known to undergo various modifications. An example of such modifications includes acetylation of tubulin. Acetylation of tubulin is deemed to promote polymerization of tubulin, thereby increasing elasticity of a microtubule, resistance of a microtubule against its collapse, and resistance of a cell against force exerted from the outside of the cell.

Acetylated tubulin detected by the method for detecting an indicator of T cell lymphoma in accordance with an embodiment of the present invention can be acetylated α-tubulin, acetylated β-tubulin, acetylated γ-tubulin, acetylated δ-tubulin, or acetylated ε-tubulin. Among these, the acetylated α-tubulin is preferable. According to the feature, it is possible to provide an accurate method for detecting an indicator of T cell lymphoma.

The acetylated tubulin can be an acetylated product of tubulin of any species, tubulin of any isoform, or a variant of these types of tubulin.

For example, human α-tubulin has been registered as "Accession Number: NM 006009. 4" in GeneBank. The acetylated tubulin can be an acetylated protein obtained by acetylation of the following protein (1) or (2). Note that an amino acid to be acetylated can be the 40th lysine counted from the amino terminus of the amino acid sequence represented by SEQ ID NO: 1:
(1) a protein constituted by the amino acid sequence represented by SEQ ID NO: 1;
(2) a protein that is constituted by the amino acid sequence represented by SEQ ID NO: 1 in which one or several amino acids are substituted, deleted, inserted, and/or added and that has microtubule-forming activity (or polymerization activity with β-tubulin).

The protein (1) above corresponds to human α-tubulin (Accession Number: NM 006009.4). Meanwhile, the protein (2) above corresponds to α-tubulin of a non-human species, an isoform of α-tubulin of a human or a non-human species, or a variant thereof. Examples of the protein (2) include tubulin α1A, tubulin α1B, tubulin α1C, tubulin α2, tubulin α3C/D, tubulin α4A, tubulin α-8-chain isoform 1, and the like.

In this specification, the phrase "one or several amino acids are substituted, deleted, inserted, and/or added" indicates, but not particularly limited to, that preferably 20 or less, more preferably 15 or less, more preferably 10 or less, more preferably 9 or less, more preferably 8 or less, more preferably 7 or less, more preferably 6 or less, more preferably 5 or less, more preferably 4 or less, more preferably 3 or less, more preferably 2 or less, most preferably 1 or less amino acid is substituted, deleted, inserted, and/or added.

Whether or not a protein has "microtubule-forming activity (or polymerization activity with β-tubulin) can be confirmed by causing the protein to express in an intended cell and detecting whether or not the protein is incorporated into a structure of a microtubule by a technique such as cell staining. If the protein can be confirmed to be incorporated into the structure of the microtubule by the method such as cell staining, the protein can be determined to have the "microtubule-forming activity (or polymerization activity with β-tubulin)".

In acetylated tubulin detected by the method for detecting an indicator of T cell lymphoma in accordance with an embodiment of the present invention, an amino acid to be acetylated and a position of the amino acid are not limited. For example, in human α-tubulin (Accession Number: NM 006009.4, in other words, a protein constituted by the amino acid sequence represented by SEQ ID NO: 1), acetylation and deacetylation occur at lysine (K40) which is located at the 40th position counted from the amino terminus. Therefore, it is preferable that acetylated tubulin detected by the method for detecting an indicator of T cell lymphoma in accordance with an embodiment of the present invention is acetylated tubulin in which the lysine (K40) is acetylated.

### (Detection of acetylated tubulin)

A specific method for detecting acetylated tubulin is not limited, provided that the step of detecting acetylated tubulin can detect acetylated tubulin.

The step of detecting acetylated tubulin can be, for example, a step of detecting acetylated tubulin by an immunological method using an anti-acetylated tubulin antibody or by mass spectrometry. The following description will discuss the individual methods.

### <A. Immunological method>

The immunological method is not particularly limited to a specific method, provided that the method detects acetylated tubulin with use of an anti-acetylated tubulin antibody. Examples of the immunological method include immunohistochemical staining, immunocytochemical staining, Western blotting, and flow cytometry. These immunological methods are known, and therefore detailed descriptions of these immunological methods are omitted here.

The anti-acetylated tubulin antibody is not particularly limited, provided that the anti-acetylated tubulin antibody specifically binds to the foregoing acetylated tubulin. Examples of the acetylated tubulin antibody include a monoclonal antibody, a polyclonal antibody, and fragments of these antibodies (e.g., Fab fragment, F(ab')₂ fragment, and single chain antibody).

The monoclonal antibody can be obtained, for example, by culturing a hybridoma that produces a monoclonal antibody against acetylated tubulin in an intended medium to obtain culture supernatant, and if necessary, purifying the culture supernatant. The hybridoma can be obtained by administering acetylated tubulin intravenously, subcutaneously, or intraperitoneally to an animal (e.g., mouse or rat), followed by obtaining an antibody-producing cell from the animal and fusing the antibody-producing cell with a myeloma cell.

The polyclonal antibody can be obtained, for example, by administering acetylated tubulin intravenously, subcutaneously, or intraperitoneally to an animal (e.g., rabbit), followed by obtaining serum from the animal and, if necessary, purifying the serum.

The Fab fragment can be obtained, for example, by digesting a monoclonal antibody against acetylated tubulin by papain and, if necessary, purifying the digested product.

The F(ab')₂ fragment can be obtained, for example, by digesting a monoclonal antibody against acetylated tubulin by pepsin and, if necessary, purifying the digested product.

The single chain antibody can be obtained, for example, by introducing into a host cell a phagemid vector containing a nucleic acid construct obtained by linking a nucleic acid encoding a variable region of a light chain of a monoclonal antibody against acetylated tubulin, a nucleic acid encoding a linker, and a nucleic acid encoding a variable region of a heavy chain of the monoclonal antibody, causing a polypeptide encoded by the nucleic acid construct to be expressed in the host cell, and, if necessary, purifying the polypeptide.

Acetylated tubulin can be detected by detecting a signal that is generated by a labeling substance that is directly bound to an anti-acetylated tubulin antibody (primary antibody) or by a labeling substance that is directly bound to a secondary antibody binding to the anti-acetylated tubulin antibody. In addition, a concentration of acetylated tubulin can be detected based on intensity of a signal generated by the labeling substance.

Examples of the labeling substance include an enzyme, an enzyme substrate, a radioisotope, a luminescent substance, and a fluorochrome.

Examples of the enzyme include peroxidase, β-galactosidase, alkaline phosphatase, glucose oxidase, acetylcholinesterase, and glucose-6-phosphate dehydrogenase. The binding of these enzymes and antibodies can be carried out by a known method using a crosslinking agent (e.g., a maleimide compound, and an N-hydroxysuccinimide ester compound).

As the enzyme substrate, a known substance can be used in accordance with the enzyme used. For example, in a case where peroxidase is used as the enzyme, orthophenylenediamine (OPD), tetramethylbenzidine (TMB), or the like is used. In a case where alkaline phosphatase is used as the enzyme, a mixed substrate of nitroblue tetrazolium (NBT) and 5-bromo-4-chloro-3-indolyl phosphatase p-toluidinyl salt (BCIP), or the like can be used.

Examples of the radioisotope include those used in general radioimmunoassay, such as ¹²⁵I, ³H, ³⁵S, and ¹⁴C.

Examples of the luminescent substance include isoluminol, acridine ester, lucigenin, and the like.

Examples of the fluorochrome include FITC (fluoresceinisothiocyanate isomer-I), HEX (6-carboxy-2',4',7',4,7,-hexafluorescein, green fluorochrome), NED (yellow fluorochrome), FAM (5-carboxyfluorescein or 6-carboxyfluorescein, blue fluorochrome), PET (red fluorochrome), VIC (green fluorochrome), LIZ (orange fluorochrome), fluorescein, rhodamine, Alexa Fluor, Cy Dye, QD (Quantum Dot), HiLyte Fluor, and the like.

### <B. Mass spectrometry>

The mass spectrometry is not particularly limited to a specific method, provided that tubulin whose mass has been increased by acetylation can be detected. The mass spectrometry can be, for example, an imaging mass spectrometry.

The imaging mass spectrometry is a method for visualizing the presence or absence and distribution of a target component on a surface of a sample by combining identification of a substance by a mass spectrometer (mass spectrometry) and observation of the sample by a microscope.

The mass spectrometry can be carried out using any technique. Specifically, the mass spectrometry can be carried out by ionization of a sample, separation of the ionized sample, and detection of the separated sample.

The method of ionizing the sample is not particularly limited. Any technique can be used in accordance with the form of the sample, and the technique can be, for example, matrix-assisted laser desorption ionization (MALDI), laser desorption ionization, sputtering (secondary ion emission), fast atom bombardment, electrospray ionization, desorption ion electrospray ionization, scanning probe electrospray ionization, atmospheric pressure chemical ionization, atmospheric pressure direct ionization, inductively coupled plasma process, electron ionization, chemical ionization, electric field desorption, or the like.

A method of separating the ionized sample is not particularly limited. Examples of the method include separation methods of time-of-flight type, magnetic field deflection type, quadrupole type, ion trap type, Fourier transform ion cyclotron resonance type, and the like.

A method of detecting the ionized sample is not particularly limited. The method can be, for example, a detection method using an electron multiplier, a microchannel plate, a Faraday cup, or the like.

In addition, the mass spectrometry can be mass spectrometry in which an arbitrary method in the above ionization methods, an arbitrary method in the above separation methods, and an arbitrary method in the above detection methods are combined as appropriate. Specifically, the mass spectrometry can be secondary ion mass spectrometry (SIMS), time of flight mass spectrometry (TOF-MS), MALDI-TOF-MS, or the like.

### [2. Test kit for T cell lymphoma]

A test kit for T cell lymphoma in accordance with an embodiment of the present invention includes an anti-acetylated tubulin antibody. Examples of the T cell lymphoma include mycosis fungoides, Sezary syndrome, adult T cell leukemia lymphoma, primary cutaneous CD30 positive lymphoproliferative disorder (e.g., anaplastic large cell lymphoma), subcutaneous panniculitis-like T cell lymphoma, and the like.

The test kit for T cell lymphoma in accordance with an embodiment of the present invention can includes an anti-acetylated tubulin antibody to which a labeling substance is bound. The test kit for T cell lymphoma in accordance with an embodiment of the present invention can include (i) an anti-acetylated tubulin antibody which is a primary antibody, and, (ii) for detecting the primary antibody, a labeling substance or a secondary antibody to which the labeling substance is bound. Note that the details of the anti-acetylated tubulin antibody have been described above, and therefore the description thereof is omitted here.

The test kit for T cell lymphoma in accordance with an embodiment of the present invention can include a secondary antibody, a chromogenic reagent, a blocking reagent, a buffer, a plate, a tube, and/or the like, which are necessary for carrying out the foregoing immunological method.

According to the test kit in accordance with an embodiment of the present invention, it is possible to quickly and simply detect acetylated tubulin.

### [3. Method for detecting indicator for evaluating inhibitory effect on T cell lymphoma]

A detection method in accordance with an embodiment of the present invention is a method for detecting an indicator for evaluating an inhibitory effect on T cell lymphoma in a subject to which a medicament has been administered, the method including the step of detecting acetylated tubulin in a T cell that has been collected from the subject to which the medicament had been administered. According to the feature, it is possible to determine whether or not a medicament administered to a subject is efficacious in treatment of T cell lymphoma. Further, the determination result can facilitate selection of (i) continuing administration of the medicament that has been administered, or (ii) discontinuing administration of the medicament that has been administered and initiating administration of another medicament.

Examples of the T cell lymphoma include mycosis fungoides, Sezary syndrome, adult T cell leukemia lymphoma, primary cutaneous CD30 positive lymphoproliferative disorder (e.g., anaplastic large cell lymphoma), subcutaneous panniculitis-like T cell lymphoma, and the like.

In this specification, the term "inhibitory effect on T cell lymphoma" means an effect of killing T cells constituting T cell lymphoma, an effect of returning T cells constituting T cell lymphoma to normal T cells, or an effect of inhibiting malignant transformation of T cells constituting T cell lymphoma (e.g., an effect of preventing or reducing expression of malignant tumor marker).

The foregoing medicament is not particularly limited, and examples thereof include an inorganic compound and an organic compound. The medicament can be used as it is, or can be used in a state of being dissolved in a solvent. Examples of the solvent include a physiological saline solution, a phosphate buffered saline, and water.

The subject is not particularly limited, and examples thereof include a human and non-human animals. The non-human animals are not particularly limited, and examples thereof include experimental animals, pets, and domestic animals. More specifically, the non-human animals can be a mouse, a rat, a rabbit, a cat, a dog, a cow, a sheep, a goat, a pig, and a horse. The subject can be a tissue (e.g., skin tissue) collected from the foregoing human or non-human animal.

A method of collecting a T cell from the subject is not particularly limited. For example, it is possible that a section of a tissue (e.g., skin tissue) is collected from the subject, and a T cell in a state of being contained in the tissue is collected.

The method of detecting acetylated tubulin in a T cell is not particularly limited, and for example, the method described in the section of <A. Immunological method> above, or the section of <B. Mass spectrometry> above can be used.

The detection method in accordance with an embodiment of the present invention can include the steps of: detecting acetylated tubulin in a T cell that has been collected from a subject to which a medicament had been administered; and then evaluating, based on the detection result, an inhibitory effect on T cell lymphoma in the subject to which the medicament had been administered.

In the evaluation step, for example, an amount of acetylated tubulin in a T cell collected from the subject to which the medicament had been administered is compared with an amount of acetylated tubulin in a T cell collected from the subject to which the medicament has not been administered yet (control). If the amount of acetylated tubulin in the T cell collected from the subject to which the medicament had been administered is smaller than the amount of acetylated tubulin in the T cell collected from the subject to which the medicament has not been administered yet, the medicament can be determined to have the inhibitory effect on T cell lymphoma. Note that the "amount of acetylated tubulin in a T cell" can be calculated as "the number of T cells expressing acetylated tubulin" or "a ratio of T cells expressing acetylated tubulin". Further, a predetermined threshold value can be set based on the control for evaluation.

### [4. Method for evaluating substance]

An evaluation method in accordance with an embodiment of the present invention is a method for evaluating whether or not a test sample is a substance having an inhibitory function against T cell lymphoma, the method including the steps of: bringing the test sample into contact with a T cell that has been collected from a subject suffering from T cell lymphoma; and detecting acetylated tubulin in the T cell which has been brought into contact with the test sample. According to the feature, therapeutic agents for T cell lymphoma can be easily screened.

Examples of the T cell lymphoma include mycosis fungoides, Sezary syndrome, adult T cell leukemia lymphoma, primary cutaneous CD30 positive lymphoproliferative disorder (e.g., anaplastic large cell lymphoma), subcutaneous panniculitis-like T cell lymphoma, and the like.

In this specification, the "inhibitory effect against T cell lymphoma" means an effect of killing T cells constituting T cell lymphoma, an effect of returning T cells constituting T cell lymphoma to normal T cells, or an effect of inhibiting malignant transformation of T cells constituting T cell lymphoma (e.g., an effect of preventing or reducing expression of malignant tumor marker).

The test sample is not particularly limited, and examples thereof include an inorganic compound, an organic compound, a plant extract, a microbial extract, and culture supernatant of various cells. The test sample can be used as it is, or can be used in a state of being dissolved in a solvent. Examples of the solvent include a physiological saline solution, a phosphate buffered saline, and water.

The subject is not particularly limited, and examples thereof include a human and non-human animals. The non-human animals are not particularly limited, and examples thereof include experimental animals, pets, and domestic animals. More specifically, the non-human animals can be a mouse, a rat, a rabbit, a cat, a dog, a cow, a sheep, a goat, a pig, and a horse. The subject can be a tissue (e.g., skin tissue) collected from the foregoing human or non-human animal.

A method of collecting a T cell from the subject is not particularly limited. For example, it is possible that a section of a tissue (e.g., skin tissue) is collected from the subject, and a T cell in a state of being contained in the tissue is collected.

The method of bringing the test sample into contact with a T cell is not particularly limited, and the T cell which has been brought into contact with the test sample can be cultured for an intended time period. Note that, during the culture, the T cell and the test sample do not need to be in contact with each other, or the T cell and the test sample can be in contact with each other. Note that the culture time is not particularly limited, and can be 1 day to 3 days, can be 1 day to 7 days, and can be 7 days or more.

The method of detecting acetylated tubulin in a T cell is not particularly limited, and for example, the method described in the section of <A. Immunological method> above, or the section of <B. Mass spectrometry> above can be used.

The evaluation method in accordance with an embodiment of the present invention can include the steps of: detecting acetylated tubulin in a T cell that has contacted a test sample; and then evaluating, based on the detection result, whether or not the test sample is a substance having an inhibitory function against T cell lymphoma.

In the evaluation step, for example, an amount of acetylated tubulin in a T cell that has been collected from a subject suffering from T cell lymphoma and that has been brought into contact with the test sample is compared with an amount of acetylated tubulin in a T cell that has been collected from a subject suffering from T cell lymphoma and that has not contacted the test sample (control). If the amount of acetylated tubulin in the T cell that has been collected from the subject suffering from T cell lymphoma and that has been brought into contact with the test sample is smaller than the amount of acetylated tubulin in the T cell that has been collected from the subject suffering from T cell lymphoma and that has not contacted the test sample, the test sample can be determined to be a substance having an inhibitory function against T cell lymphoma. Note that the "amount of acetylated tubulin in a T cell" can be calculated as "the number of T cells expressing acetylated tubulin" or "a ratio of T cells expressing acetylated tubulin". Further, a predetermined threshold value can be set based on the control for evaluation. Note that the "subject suffering from T cell lymphoma" can be the same subject or can be different subjects. From the viewpoint of more accurately evaluating the substance, the subject is preferably the same subject.

### [5. Therapeutic agent for T cell lymphoma]

A therapeutic agent for T cell lymphoma in accordance with an embodiment of the present invention contains, as an active ingredient, an agent that inhibits polymerization of tubulin; an agent that inhibits acetylation of tubulin, and/or an agent that inhibits aggregation of acetylated tubulin.

Examples of the agent that inhibits polymerization of tubulin include ASMP-3 (product name: Ansamitocin P-3, Actinosynnema pretiosum), Demecolcine (product name: Colcemid), Colchicine (product name: Colchicine, Colchicum autumnale), Diminisher of microtubule; 2-(1R-Isopropyl-2-hydroxyethylamino)-6-(3-aminophenylthio)-9-isopropylpurine; NG72 (product name: Diminutol), (5S)-1-[(2S)-O-(N,N-Val-Val-N-Me-Val-Pro-Pro)-2-hydroxyisovaleryl]-2-oxo-4-methoxy-5-benzyl-3-pyrroline (product name: Dolastatin 15), NSC 698666 (product name: Indanocine), 10-[(3-Hydroxy-4-methoxybenzylidene)]-9(10H)-anthracenone (product name: Tubulin Polymerization Inhibitor), (E)-1,3-Dihydro-6-methoxy-3-(3,4,5-trimethoxybenzylidene)-1H-indol-2-one (product name: Tubulin Polymerization Inhibitor II), combrestatin, 2-methoxyestradiol, methoxy benzenesulfonamides, vinblastine, vincristine, vinorelbine, vinfluine, dolastatins, halichondrins, hemiasterlins, and cryptophysin 52.

Examples of the agent that inhibits acetylation of tubulin include N-(1H-Benzotriazol-1-yl)-2,4-dichlorobenzamide;1-(2,4-Dichlorobenzoyl)-1H-benzotriazole (product name: ITSA1) and an inhibitor for alpha-tubulin N-acetyltransferase (ATAT1).

An amount of the active ingredient contained in the therapeutic agent in accordance with an embodiment of the present invention is not particularly limited, and can be preferably 0.001% by mass to 100% by mass, 0.01% by mass to 100% by mass, 0.1% by mass to 100% by mass, 0.1% by mass to 95% by mass, 0.1% by mass to 90% by mass, 0.1% by mass to 80% by mass, 0.1% by mass to 70% by mass, 0.1% by mass to 60% by mass, 0.1% by mass to 50% by mass, 0.1% by mass to 40% by mass, 0.1% by mass to 30% by mass, 0.1% by mass to 20% by mass, 0.1% by mass to 10% by mass, with respect to 100% by mass of the therapeutic agent.

The therapeutic agent for T cell lymphoma can contain other components as long as such other components do not alter the therapeutic function of the therapeutic agent. Examples of the other components include a pharmaceutically acceptable component, for example, a buffer, a pH adjuster, an isotonizing agent, a preservative, an excipient, a carrier, a diluent, a solvent, a solubilizer, a stabilizer, an antioxidant, a high molecular weight polymers, a filler, a binder, a surfactant, a stabilizer, and the like.

Examples of the buffer include phosphoric acid, phosphate, boric acid, borate, citric acid, citrate, acetic acid, acetate, carbonic acid, carbonate, tartaric acid, tartrate, ε-aminocaproic acid, Trometamol, and the like. Examples of the phosphate include sodium phosphate, sodium dihydrogen phosphate, disodium hydrogen phosphate, potassium phosphate, potassium dihydrogen phosphate, dipotassium hydrogen phosphate, and the like. Examples of the borate include borax, sodium borate, potassium borate, and the like. Examples of the citrate include sodium citrate, disodium citrate, trisodium citrate, and the like. Examples of the acetate include sodium acetate, potassium acetate, and the like. Examples of the carbonate include sodium carbonate, sodium hydrogencarbonate, and the like. Examples of the tartrate include sodium tartrate, potassium tartrate, and the like.

Examples of the pH adjuster include hydrochloric acid, phosphoric acid, citric acid, acetic acid, sodium hydroxide, potassium hydroxide, and the like.

Examples of the isotonizing agent include ionic isotonizing agents (such as sodium chloride, potassium chloride, calcium chloride, magnesium chloride), and nonionic isotonising agents (such as glycerin, propylene glycol, sorbitol, mannitol).

Examples of the preservative include benzalkonium chloride, benzalkonium bromide, benzethonium chloride, sorbic acid, potassium sorbate, methyl parahydroxybenzoate, propyl parahydroxybenzoate, chlorobutanol, and the like.

Examples of the antioxidants include ascorbic acid, tocophenol, dibutylhydroxytoluene, butyl hydroxyanisole, sodium erythorbate, propyl gallate, sodium sulfite, and the like.

Examples of the high molecular weight polymer include methylcellulose, ethylcellulose, hydroxymethylcellulose, hydroxy ethylcellulose, hydroxypropylcellulose, hydroxyethylmethylcellulose, hydroxy propylmethylcellulose, carboxymethylcellulose, sodium carboxymethylcellulose, hydroxypropylmethylcellulose acetate succinate, hydroxypropylmethylcellulose phthalate, carboxymethylethylcellulose, cellulose acetate phtalate, polyvinylpyrrolidone, polyvinyl alcohol, a carboxyvinyl polymer, polyethylene glycol, and the like.

Examples of the carrier, excipient and diluent include lactose, dextrose, sucrose, sorbitol, mannitol, xylitol, erythritol, maltitol, starch, acacia rubber, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oil, and the like.

Examples of the solvent and the solubilizer include glycerin, DMSO, DMA, N-methylpyrrolidone, ethanol, benzyl alcohol, isopropyl alcohol, polyethylene glycols of various molecular weights (such as PEG300 and PEG400), propylene glycol, and the like.

Examples of the surfactant include nonionic, anionic, cationic, zwitterionic, polymeric, and amphoteric surfactants.

Examples of the stabilizer include fatty acid, fatty alcohol, alcohol, long chain fatty ester, long chain ether, a hydrophilic derivative of fatty acid, polyvinylpyrrolidone, polyvinyl ether, polyvinyl alcohol, hydrocarbon, a hydrophobic polymer, a hygroscopic polymer, and an amide analogue.

Further, the above other components can be contained solely or in combination of two or more types thereof.

An amount of the above other components is not particularly limited, and can be 0% by mass to 99.999% by mass, 0% by mass to 99.99% by mass, 0% by mass to 99.9% by mass, 5% by mass to 99.9% by mass, 10% by mass to 99.9% by mass, 20% by mass to 99.9% by mass, 30% by mass to 99.9% by mass, 40% by mass to 99.9% by mass, 50% by mass to 99.9% by mass, 60% by mass to 99.9% by mass, 70% by mass to 99.9% by mass, 80% by mass to 99.9% by mass, 90% by mass to 99.9% by mass, with respect to 100% by mass of the therapeutic agent.

An administration form of the therapeutic agent for T cell lymphoma is not particularly limited, and examples thereof include an oral agent, a parenteral agent, and a liniment.

Examples of cancers to which the therapeutic agent can be applied include mycosis fungoides, Sezary syndrome, adult T cell leukemia lymphoma, primary cutaneous CD30 positive lymphoproliferative disorder (e.g., anaplastic large cell lymphoma), subcutaneous panniculitis-like T cell lymphoma, and the like.

### [6. Other]

An aspect of the present invention can also be featured as below:
[1] A method for detecting an indicator of T cell lymphoma in accordance with an aspect of the present invention includes the step of detecting acetylated tubulin in a T cell that has been collected from a subject.
[2] According to the method for detecting an indicator of T cell lymphoma in accordance with an aspect of the present invention, in the step of detecting acetylated tubulin, the acetylated tubulin is detected by an immunological method using an anti-acetylated tubulin antibody or by mass spectrometry.
[3] A test kit for T cell lymphoma in accordance with an aspect of the present invention includes an anti-acetylated tubulin antibody.
[4] A detection method in accordance with an aspect of the present invention is a method for detecting an indicator for evaluating an inhibitory effect on T cell lymphoma in a subject to which a medicament has been administered, the method including the step of detecting acetylated tubulin in a T cell that has been collected from the subject to which the medicament had been administered.
[5] An evaluation method in accordance with an aspect of the present invention is a method for evaluating whether or not a test sample is a substance having an inhibitory function against T cell lymphoma, the method including the steps of: bringing the test sample into contact with a T cell that has been collected from a subject suffering from T cell lymphoma; and detecting acetylated tubulin in the T cell which has been brought into contact with the test sample.
[6] A therapeutic agent for T cell lymphoma in accordance with an aspect of the present invention contains, as an active ingredient, an agent that inhibits aggregation of acetylated tubulin, and/or an agent that inhibits acetylation of tubulin.

An aspect of the present invention can also be featured as below:
<1> A method for treating T cell lymphoma, including the step of administering, to a subject (e.g., a human or a non-human animal), a therapeutic agent for T cell lymphoma, the therapeutic agent containing, as an active ingredient, an agent that inhibits aggregation of acetylated tubulin, and/or an agent that inhibits acetylation of tubulin.
<2> Use of an agent that inhibits aggregation of acetylated tubulin and/or an agent that inhibits acetylation of tubulin, for producing a therapeutic agent for T cell lymphoma.

### Examples

The following description will discuss an Example of the present invention. The present invention is not limited to Example described below, but can be altered in various ways within the scope of the present invention.

### [Expression pattern analysis]

### (Collection, mounting)

Skin from a lesion and skin from a normal part of a subject (patient) suffering from T cell lymphoma were collected. Paraffin-embedded samples were prepared from the skins. The paraffin-embedded samples were deparaffinized using xylene. Then, the deparaffinized samples were immersed in 100% ethanol, 95% ethanol, and purified water for 10 minutes each, and were thus gradually hydrated.

### (Antigen activation, permeabilization)

Subsequently, the hydrated samples were boiled in 1 mM ethylenediamine tetra acetic acid (EDTA) aqueous solution for 15 minutes to activate the antigen. The samples were then blocked and permeabilized by incubation in a blocking/permeabilizing agent (10% serum and 0.1 w/w% polyoxyethylene sorbitan monolaurate-containing phosphate buffered saline (PBS) solution-containing reagent) for 30 minutes.

### (Immunostaining)

In the samples, the following primary antibodies were used to detect acetylated tubulin, langerin, CCR4, and CD4, respectively.
- Anti-acetylated tubulin antibody [available from Sigma-Aldrich, product name: MONOCLONAL ANTI-ACETYLATED TUBULIN CLONE 6-11B-1, product number: T6793],
- Anti-langerin antibody [available from abcam, product name: Anti-Langerin [EPR15863] antibody, catalogue number: ab192027],
- Anti-CCR4 antibody [available from abcam, product name: Anti-CCR4 antibody, catalogue number: ab1669],
- Anti-CD4 antibody [available from Proteintech, product name: CD4 Polyclonal ANTIBODY, catalogue number: 19068-1-AP].

These antibodies and the samples were allowed to react overnight at 4°C. In addition, concentrations of these antibodies used and reaction methods were in conformity to respective protocols. After the reaction, the samples were cleaned using a cleaning solution A [phosphate buffered saline (PBS) solution containing 0.1 w/w% polyoxyethylene sorbitan monolaurate].

In order to detect the above primary antibodies, the following secondary antibodies were used:
- Fluorochrome-labeled anti-rabbit IgG antibody [available from abcam, product name: Goat Anti-Rabbit IgG H8vL (fluorochrome: Alexa Fluor (registered trademark) 594), catalogue number: ab150080];
- Fluorochrome-labeled anti-goat IgG antibody [available from abcam, product name: Donkey Anti-Goat IgG H8vL (fluorochrome: Alexa Fluor (registered trademark) 594), catalogue number: ab150132];
- Fluorochrome-labeled anti-mouse IgG antibody [available from abcam, product name: Goat Anti-Mouse IgG H8vL (fluorochrome: Alexa Fluor (registered trademark) 488, catalogue number: ab150113)].

These antibodies and the samples were allowed to react overnight at 4°C. In addition, concentrations of these antibodies used and reaction methods were in conformity to respective protocols.

### (Nuclear staining)

The nuclei were stained with use of the following nuclear staining agent.
- Nuclear staining agent Hoechst 33342 [available from Thermo Fisher Scientific, item name: Hoechst 33342, Trihydrochloride, Trihydrate-10 mg/mL Solution in Water, product number: H3570].

The nuclear staining agent and the samples were allowed to react overnight at 4°C. A concentration used and reaction were in conformity to protocols of the nuclear staining agent.

### (Mounting)

The obtained samples were cleaned using the cleaning solution A, and then mounted using the following antifade mountant to obtain samples for microscopic observation.
- Antifade mountant, [available from Thermo Fisher Scientific, product name: ProLong (registered trademark) Gold antifade mountant, product number: P36934].

### (Microscopic observation)

With use of a confocal microscope [available from Olympus Corporation, product number: FV1200], a Langerhans cell, a CCR4 positive cell, and a CD4 positive cell contained in each of the samples were detected, and expression patterns of cell-surface markers in acetylated tubulin positive cells were analyzed. In the microscopic observation, imaging was carried out while switching fluorescent filters to record the respective types of fluorescence. The results are shown in Fig. 1.

### [Results]

In Fig. 1, (a) shows microscopic images of an expression pattern with the Langerhans cell marker and acetylated tubulin (Ac-Tub); (b) shows microscopic images of an expression pattern with the CCR4 cell marker and acetylated tubulin (Ac-Tub); (c) shows microscopic images of an expression pattern with the CD4 cell marker and acetylated tubulin (Ac-Tub); and (d) shows microscopic images of an expression pattern with the CD8 cell marker and acetylated tubulin (Ac-Tub).

As shown in (a) of Fig. 1, cells expressing acetylated tubulin were not observed in the Langerhans cells, which are a type of dendritic cells.

As shown in (c) and (d) of Fig. 1, expression of acetylated tubulin was observed in many of CD4⁺ T cells and CD8⁺ T cells which are less malignant. In addition, as shown in (b) of Fig. 1, expression of acetylated tubulin was observed in approximately 100% of cells in CCR4⁺ T cells which are highly malignant. Since expression of acetylated tubulin was observed in both the less malignant CD4⁺ T cells and CD8⁺ T cells and the highly malignant CCR4⁺ T cells, it has been found that acetylated tubulin can be used as an indicator for diagnosing not only high-malignancy-grade (late-stage) T cell lymphoma but also low-malignancy-grade (early-stage) T cell lymphoma.

### Industrial Applicability

The present invention can be widely utilized in the medical field, and in particular, can be suitably utilized for methods of detecting an indicator of T cell lymphoma, test kits, treatments, and the like.

## Claims

1. A method for detecting an indicator of T cell lymphoma, said method comprising the step of:
detecting acetylated tubulin in a T cell that has been collected from a subject.

2. The method as set forth in claim 1, wherein:
in the step of detecting acetylated tubulin, the acetylated tubulin is detected by an immunological method using an anti-acetylated tubulin antibody or by mass spectrometry.

3. A test kit for T cell lymphoma, said test kit comprising an anti-acetylated tubulin antibody.

4. A method for detecting an indicator for evaluating an inhibitory effect on T cell lymphoma in a subject to which a medicament has been administered, said method comprising the step of:
detecting acetylated tubulin in a T cell that has been collected from the subject to which the medicament had been administered.

5. A method for evaluating whether or not a test sample is a substance having an inhibitory function against T cell lymphoma, said method comprising the steps of:
bringing the test sample into contact with a T cell that has been collected from a subject suffering from T cell lymphoma; and
detecting acetylated tubulin in the T cell which has been brought into contact with the test sample.

6. A therapeutic agent for T cell lymphoma, said therapeutic agent comprising, as an active ingredient,
an agent that inhibits aggregation of acetylated tubulin, and/or
an agent that inhibits acetylation of tubulin.
